# EUROPEAN PATENT APPLICATION

(11) **EP 2 578 256 A1**
(43) Date of publication of application: **10.04.2013**
(21) Application number: 11184096.3
(22) Date of filing: 06.10.2011
(51) Int. Cl.: A61M 5/32

(54) **Needle safety device**

(71) Applicant: Sanofi-Aventis Deutschland GmbH, 65929 Frankfurt (DE)
(72) Inventor: The designation of the inventor has not yet been filed

(57) **Abstract**

Described is a needle safety device (1, 101, 201) comprising a needle hub (1.2, 101.2, 201.2) including a latch element (1.2.2, 101.2.2, 201.2.2), a needle (1.3, 101.3, 201.3) coupled to the needle hub (1.2, 101.2, 201.2) and having a distal tip (1.3.1, 101.3.1, 201.3.1), and a needle shield (1.1, 101.1, 201.1) telescopically coupled to the needle hub (1.2, 101.2, 201.2). The needle shield (1.1, 101.1, 201.1) includes a resilient arm (1.1.2, 101.1.2, 201.1.2) adapted to engage the latch element (1.2.2, 101.2.2, 201.2.2). When the needle shield (1.1, 101.1, 201.1) is in a first axial position (PA1, PA101, PA201) relative to the needle hub (1.2, 101.2, 201.2), the needle shield (1.1, 101.1, 201.1) covers the distal tip (1.3.1, 101.3.1, 201.3.1) of the needle (1.3, 101.3, 201.3). When the needle shield (1.1, 101.1, 201.1) is in a retracted position (PR1, PR101, PR201) relative to the needle hub (1.2, 101.2, 201.2), the needle shield (1.1, 101.1, 201.1) is retracted to expose the distal tip (1.3.1, 101.3.1, 201.3.1) of the needle (1.3, 101.3, 201.3) and the arm (1.1.2, 101.1.2, 201.1.2) is deflected radially. When the needle shield (2.1) is in a second axial position (PA2) relative to the needle hub (1.2, 101.2, 201.2), the needle shield (1.1, 101.1, 201.1) covers the distal tip (1.3.1, 101.3.1, 201.3.1) of the needle (1.3, 101.3, 201.3) and the arm (1.1.2, 101.1.2, 201.1.2) engages the latch element (1.2.2, 101.2.2, 201.2.2) to prevent proximal movement of the needle shield (1.1, 101.1, 201.1) relative to the needle hub (1.2, 101.2, 201.2).

## Description

### Background of the Invention

Medicament delivery devices (e.g., pen injectors, syringes, auto-injectors, etc.) that contain a selected dosage of a medicament are well known devices for administering the medicament to a patient. Safety devices for covering a needle of the delivery device before and after use are also well known. Typically, a needle shield of the safety device is either manually moved or automatically to surround the medical needle. Various attempts have been made to develop an optimally sized and functioning safety device. However, there remains a need for an optimal needle safety device.

### Summary of the Invention

It is an object of the present invention to provide an improved needle safety device that minimizes the risk of an accidental needle stick injury, that is safe to handle, and that provides needle safety before and after the medicament is delivered.

In an exemplary embodiment, a needle safety device according to the present invention comprises a needle hub including a latch element, a needle coupled to the needle hub and having a distal tip, and a needle shield telescopically coupled to the needle hub. The needle shield includes a resilient arm adapted to engage the latch element. When the needle shield is in a first axial position relative to the needle hub, the needle shield covers the distal tip of the needle. When the needle shield is in a retracted position relative to the needle hub, the needle shield is retracted to expose the distal tip of the needle and the arm is deflected radially. When the needle shield is in a second axial position relative to the needle hub, the needle shield covers the distal tip of the needle and the arm engages the latch element to prevent proximal movement of the needle shield relative to the needle hub.

In an exemplary embodiment, the needle hub includes a retaining element adapted to engage a slot on the needle shield to prevent distal movement of the needle shield relative to the needle hub in the first axial position.

In an exemplary embodiment, the needle shield includes a cover element defining a cavity for accommodating deflection of the arm.

In an exemplary embodiment, the latch element includes a resilient guide formed in a recess. A proximal end of the arm includes a hook having a first ramped surface adapted to deflect the guide when the needle shield moves from the retracted position to the second axial position. When the guide is deflected, the hook engages the recess to lock the needle shield in the second axial position.

In an exemplary embodiment, the arm includes an aperture adapted to engage the latch element. The aperture includes a first section having a first width and a second section having a second width. A third section of the aperture has a third width. The first width and the third width are substantially equal. Resilient stop elements are formed between the second section and the third section. The latch element is T-shaped and has a cross section substantially equal to the second width and a stem section substantially equal to the first width. The cross section of the latch element engages the arm and causes the arm to deflect radially when the needle shield is moved from the first axial position to the retracted position. The latch element engages the aperture when the cross section reaches the second section when the needle shield is moved from the first axial position to the retracted position. The stem portion causes the stop elements to deflect when the needle shield moves from the retracted position to the second axial position. When the latch element is in the third section, the stop elements return to a non-deflected position to lock latch element in the third section and lock the needle shield in the second axial position. The cross section abuts the arm in the first axial position. The latch element engages the aperture when needle shield is moved from the retracted position to the second axial position. The stem portion causes a narrowed portion of the aperture to deflect when latch element moves from the first section to the second section. When the latch element is in the second section, the narrowed portion of the aperture returns to a non-deflected position to lock latch element in the second section and lock the needle shield in the second axial position.

In an exemplary embodiment, a biasing element applies a biasing force to the needle shield in a distal direction when the needle shield is in the retracted position.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given herein below and the accompanying drawings which are given by way of illustration only, and thus, are not limited of the present invention, and wherein:
- Figure 1: shows an exploded view of an exemplary embodiment of a needle safety device according to the present invention.
- Figure 2: shows a top view of the exemplary embodiment of a needle safety device shown in Figure 1.
- Figure 3: shows a perspective view of a sectioning of the exemplary embodiment of a needle safety device shown in Figure 1 before use.
- Figure 4: shows a sectional view of the exemplary embodiment of a needle safety device shown in Figure 1.
- Figure 5: shows a sectional view of the exemplary embodiment of a needle safety device shown in Figure 1 during use.
- Figure 6: shows a perspective view of a sectioning of the exemplary embodiment of a needle safety device shown in Figure 1 during use.
- Figure 7: shows a sectional view of the exemplary embodiment of a needle safety device shown in Figure 1 during use.
- Figure 8: shows a perspective view of a sectioning of the exemplary embodiment of a needle safety device shown in Figure 1 after use.
- Figure 9: shows a perspective view of a sectioning of the exemplary embodiment of a needle safety device shown in Figure 1 after use.
- Figure 10: shows an exploded view of another exemplary embodiment of a needle safety device according to the present invention.
- Figure 11: shows a top view of the exemplary embodiment of the needle safety device shown in Figure 10.
- Figure 12: shows a perspective view of the exemplary embodiment of the needle safety device shown in Figure 10 before use.
- Figure 13: shows a sectional view of the exemplary embodiment of the needle safety device shown in Figure 10 before use.
- Figure 14: shows a sectional view of the exemplary embodiment of the needle safety device shown in Figure 10 during use.
- Figure 15: shows a perspective view of the exemplary embodiment of the needle safety device shown in Figure 10 during use.
- Figure 16: shows a sectional view of the exemplary embodiment of the needle safety device shown in Figure 10 during use.
- Figure 17: shows a sectional view of the exemplary embodiment of the needle safety device shown in Figure 10 after use.
- Figure 18: shows a perspective view of the exemplary embodiment of the needle safety device shown in Figure 10 after use.
- Figure 19: shows an exploded view of yet another exemplary embodiment of a needle safety device according to the present invention.
- Figure 20: shows a sectional view of an exemplary embodiment of a needle shield of the exemplary embodiment of the needle safety device shown in Figure 19.
- Figure 21: shows a sectional view of an exemplary embodiment of a needle shield of the exemplary embodiment of the needle safety device shown in Figure 19.
- Figure 22: shows a perspective view of an exemplary embodiment of a needle shield of the exemplary embodiment of the needle safety device shown in Figure 19.
- Figure 23: shows a top view of the exemplary embodiment of the needle safety device shown in Figure 19.
- Figure 24: shows a perspective view of the exemplary embodiment of the needle safety device shown in Figure 19 before use.
- Figure 25: shows a sectional view of the exemplary embodiment of the needle safety device shown in Figure 19 before use.
- Figure 26: shows a perspective view of a sectioning of the exemplary embodiment of the needle safety device shown in Figure 19 during use.
- Figure 27: shows a sectional view of the exemplary embodiment of the needle safety device shown in Figure 19 during use.
- Figure 28: shows a perspective view of a sectioning of the exemplary embodiment of the needle safety device shown in Figure 19 during use.
- Figure 29: shows a sectional view of the exemplary embodiment of the needle safety device shown in Figure 19 during use.
- Figure 30: shows a perspective view of a sectioning of the exemplary embodiment of the needle safety device shown in Figure 19 after use.
- Figure 31: shows a sectional view of a sectioning of the exemplary embodiment of the needle safety device shown in Figure 19 after use.

Corresponding parts are marked with the same reference symbols in all figures.

### Detailed Description

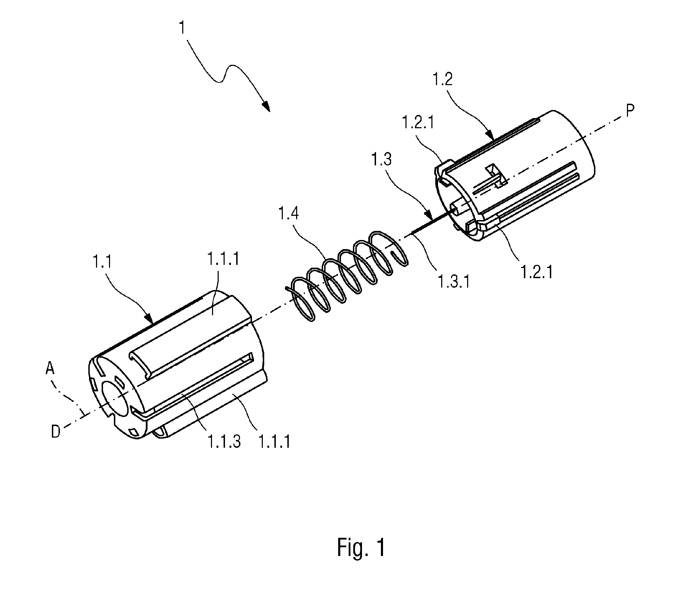

Figure 1 shows an exploded view of an exemplary embodiment of a needle safety device 1 according to the present invention. The needle safety device 1 comprises a needle shield 1.1 telescopically coupled to a needle hub 1.2 having a needle 1.3, and a biasing element 1.4 applying a biasing force to the needle shield 1.1 relative to the needle hub 1.2. Further, the needle safety device 1 includes a longitudinal axis A extending through from a proximal direction P to a distal direction D.

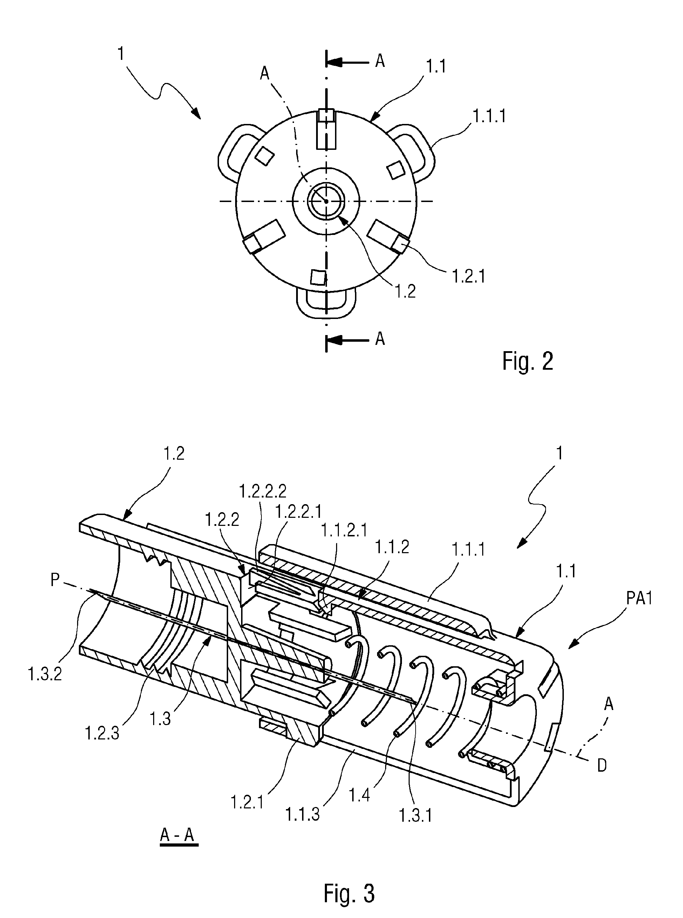

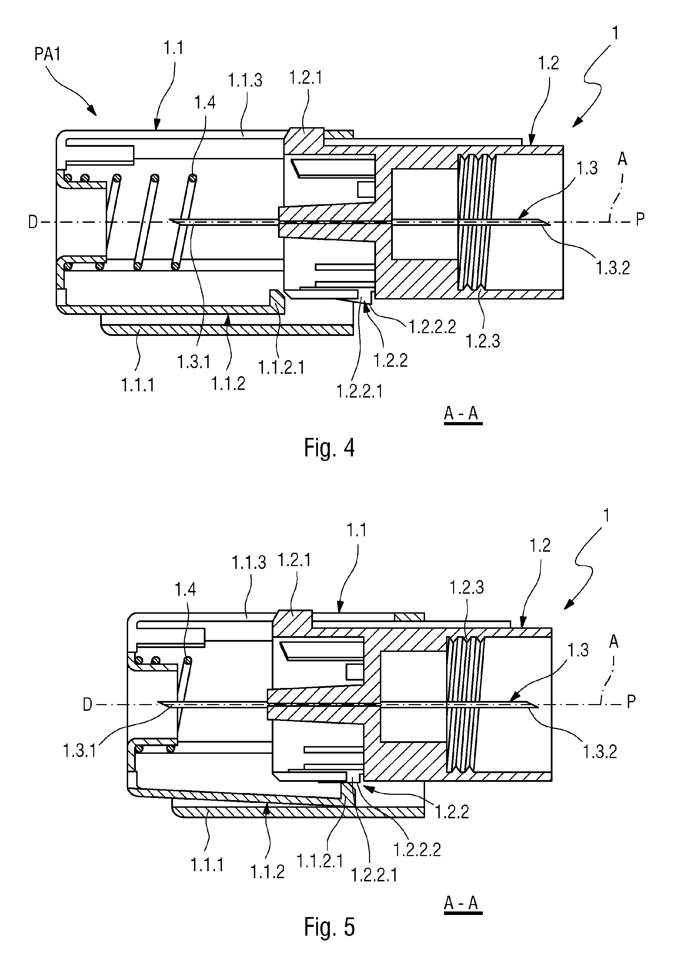

In an exemplary embodiment, the needle shield 1.1 includes one or more cover elements 1.1.1 formed integrally with an outer circumference of the needle shield 1.1 and one or more slots 1.1.3. As shown in Figures 3 and 4, the cover element 1.1.1 at least partially covers a resilient arm 1.1.2 having a hook 1.1.2.1 on its proximal end. In use, as described further below, the arm 1.1.2 may deflect radially into a cavity within the cover 1.1.1.

The slots 1.1.3 are adapted to engage retaining elements 1.2.1 formed on a distal end of the needle hub 1.2. In use, as described further below, the retaining elements 1.2.1 I translate within the slots 1.1.3 to prevent removal of the needle shield 1.1 from the needle hub 1.2, limit movement of the needle shield 1.1 relative to the needle hub 1.2 in a proximal direction, and prevent rotation of the needle shield 1.1 relative to the needle hub 1.2.

Figures 3 and 4 show the exemplary embodiment of the needle safety device 1 shown in Figure 1 in a first axial position (PA1). In the exemplary embodiment, the needle hub 1.2 comprises a thread 1.2.3 for mounting the needle safety device 1 to a not shown medicament delivery device. Alternatively, the needle hub 1.2 may be attached to the delivery device by other suitable couplings, like for example bayonet type couplings or snap-fit couplings. The delivery device can be designed as a pen injector or according to alternative embodiments, the delivery device may be designed as a syringe, a dental syringe, an auto-injector or a similar device suitable for delivering a medicament to a patient.

The needle 1.3 is mounted to the needle hub 1.2. In the exemplary embodiment shown, the needle 1.3 is arranged as a double pointed needle with a pointed distal tip 1.3.1 and a pointed proximal tip 1.3.2. The pointed proximal tip 1.3.2 is adapted to be inserted into a cartridge or container containing the medicament that is retained in the delivery device when the needle safety device 1 is coupled to the delivery device.

In the first axial position (PA1), the needle shield 1.1 is in an advanced axial position relative to the needle hub 1.2 such that the needle shield 1.1 covers the distal tip 1.3.1 I of the needle 1.3. The needle shield 1.1 is prevented from moving proximally relative to the needle hub 1.2 when in the first axial position (PA1) by the retaining elements 1.2.1 which abut a proximal end of the slots 1.1.3.

In an exemplary embodiment, the biasing element 1.4 is adapted to bias the needle shield 1.1 with respect to the needle hub 1.2 in the first axial position (PA1) and arranged as a compression spring which bears against the needle shield 1.1 in the distal direction D and against the needle hub 1.2 in a proximal direction P. That is, in the first axial position (PA1), the biasing element 1.4 may be preloaded. In another exemplary embodiment, there may be no load on the biasing element 1.4 in the first axial position (PA1).

In an exemplary embodiment, the needle hub 1.2 further comprises at least one latch element 1.2.2 in axial alignment with the arm 1.1.2. In the exemplary embodiment, the latch element 1.2.2 includes a distal ramped portion, a recess 1.2.2.1 and a resilient guide 1.2.2.2 in the recess 1.2.2.1. The resilient guide 1.2.2.2 is adapted to deflect at least circumferentially within the recess 1.2.2.1.

In an exemplary embodiment, the hook 1.2.1.1 on the arm 1.2.1 is adapted to engage the distal ramped portion of the latch element 1.2.2 when the needle shield 1.1 moves proximally relative to the needle hub 1.2. The hook 1.2.1.1 may include a first ramped portion to engage the distal ramped portion of the latch element 1.2.2 to deflect the arms 1.2.1 when the needle shield 1.1 moves proximally relative to the needle hub 1.2. The hook 1.2.1.1 may further include a second ramped portion to engage the guide 1.2.2.2 and cause it to deflect circumferentially within the recess 1.2.2.1 so that the hook 1.2.1.1 can engage the recess 1.2.2.1. The second ramped portion may lie in a different plane than the first ramped portion. In another exemplary embodiment, the hook 1.2.1.1 may include only the first ramped portion which may be disposed at an angle to engage the guide 1.2.2.2 and the distal ramped portion of the latch element 1.2.2.

Those of skill in the art will understand that the number of arms 1.2.1 may correspond to the number of latch elements 1.2.2, and the number of retaining elements 1.2.1 may correspond to the number of slots 1.1.3.

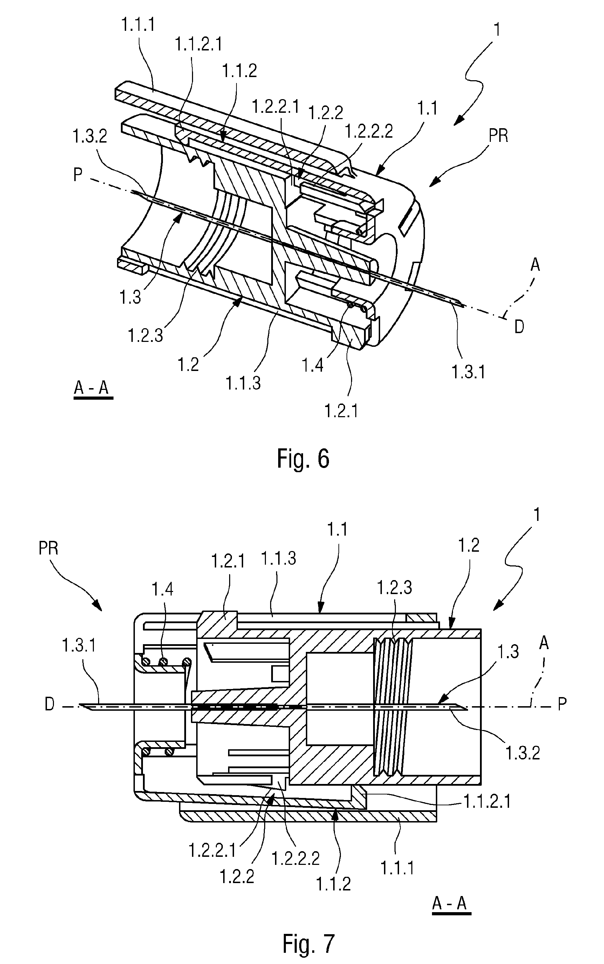

Figures 5, 6 and 7 show the exemplary embodiment of the needle safety device 1 in a retracted position (PR) in which the needle shield 1.1 has moved proximally relative to the needle hub 1.2 such that the distal tip 1.3.1 of the needle 1.3 is exposed. The needle safety device 1 may attain the retracted position (PR) when the needle safety device 1 is pressed against an injection site. In the retracted position (PR), the biasing element 1.4 may be compressed.

When the needle shield 1.1 begins to move proximally relative to the needle hub 1.2, the first ramped portion of the hook 1.1.2.1 may engage the distal ramped portion of the latch element 1.2.2, causing the arm 1.1.2 to deflect radially away from the axis A and into the cavity of the cover element 1.1.1. As the needle shield 1.1 moves further proximally relative to the needle hub 1.2, the hook 1.1.2.1 may pass proximally over the recess 1.2.2.1 due to the presence of the guide 1.2.2.2 which obstructs access to the recess 1.2.2.1. The needle shield 1.1 attains the retracted position (PR) when the retaining element 1.2.1 abuts a distal portion of the slot 1.1.3.

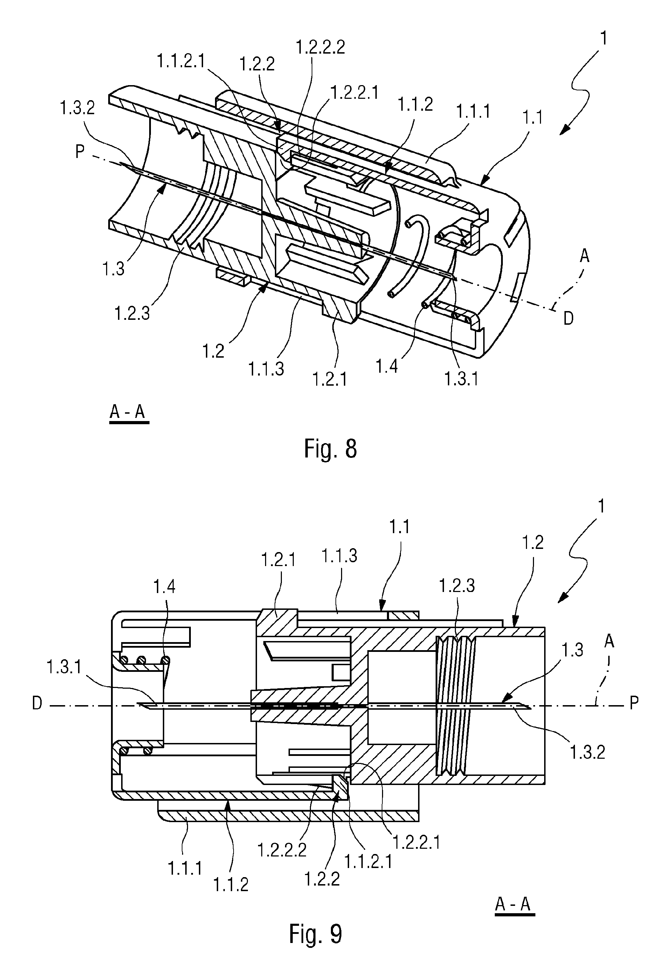

Figures 8 and 9 show the exemplary embodiment of the needle safety device 1 in a second axial position (PA2) in which the needle shield 1.1 has moved distally relative to the needle hub 1.2 such that the distal tip 1.3.1 of the needle 1.3 is covered. When the needle shield 1.1 moves distally from the retracted position (PR), under the force of the biasing element 1.4, the second ramp on the hook 1.1.2.1 engages the guide 1.2.2.2, causing the guide 1.2.2.2 to deflect circumferentially within the recess 1.2.2.1. When the guide 1.2.2.2 is deflected, sufficient space is available in the recess 1.2.2.1 so that the hook 1.1.2.1 returns to its non-deflected state and engages the recess 1.2.2.1. When the hook 1.1.2.1 has engaged the recess 1.2.2.1, the needle shield 1.1 is prevented from proximal and distal movement relative to the needle hub 1.2.

Hence the engagement of the hook 1.1.2.1 and the recess 1.2.2.1 introduces a no-return feature so that needle safety device 1 may only be used once and needle stick injuries with contaminated needles may be avoided. The safety needle device 1 according to the first embodiment is irreversibly locked in the second advanced position (PA2) and may be safely detached from the delivery device and disposed after use.

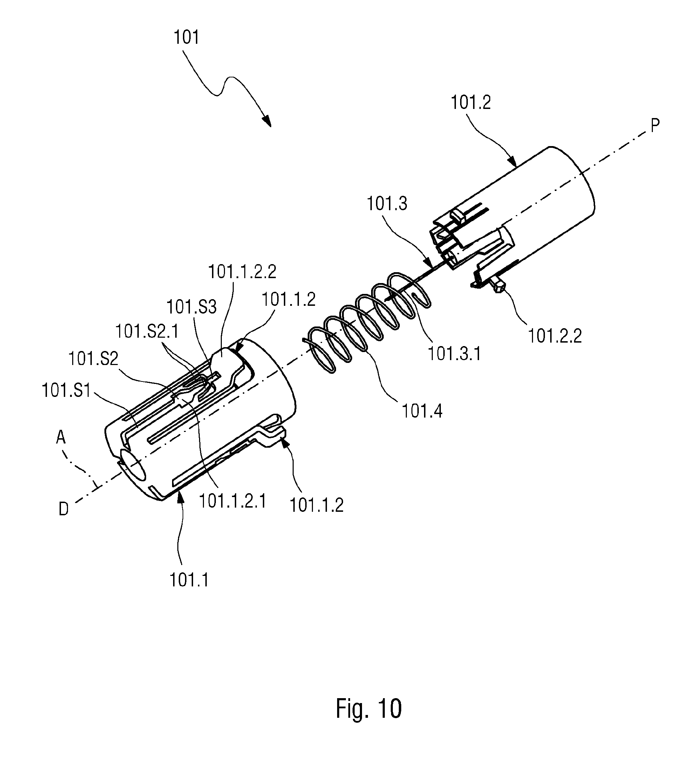

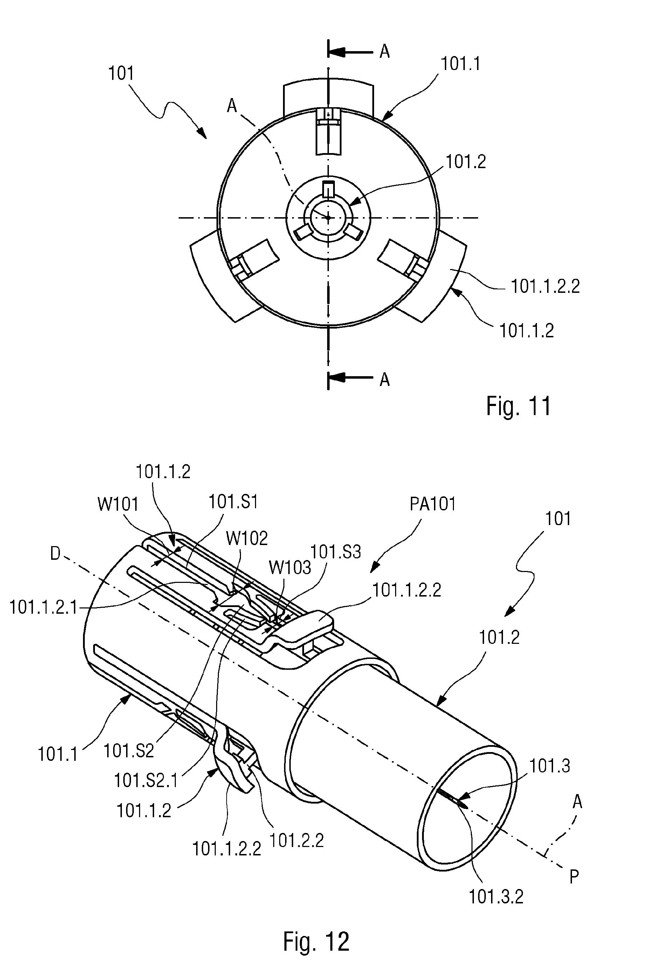

Figures 10, 11 and 12 show another exemplary embodiment of a needle safety device 101 according to the present invention. The needle safety device 101 comprises a needle shield 101.1 telescopically coupled to a needle hub 101.2 having a needle 101.3, and a biasing element 101.4 applying a biasing force to the needle shield 101.1 relative to the needle hub 101.2. Further, the needle safety device 101 includes a longitudinal axis A extending through from a proximal direction P to a distal direction D.

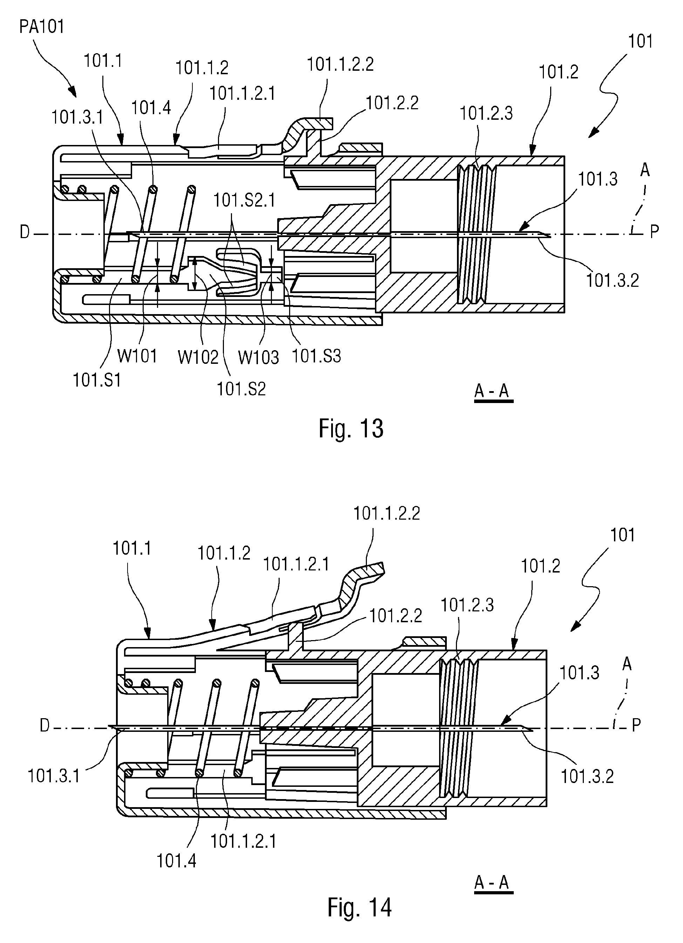

In an exemplary embodiment, the needle shield 101.1 includes a plurality of resilient arms 101.2 having an aperture 101.1.2.1 with a number of different width sections 101.S1, 101.S2 to 101.S3 in a longitudinal direction parallel to the axis A. As shown in Figure 13, the first section 101 .S1 may be formed at a distal end of the aperture 101.1.2.1 and have a first width W101. The second section 101.S2 may be formed proximal of the first section 101 .S1 and have a second width W102 greater than the first width W101 of the first section 101.S1. The third section 101.S3 may be formed proximal of the second section 101.S2 and have a third width W103 equal to the first width W101. Two resilient stop elements 101.S2.1 may be formed between the second section 101.S2 and the third section 101.S3. The stop elements 101.S2.1 may be angled toward an axis of the aperture 101.1.2.1 and capable of deflecting radially away from the axis of the aperture 101.1.2.1.

Referring back to Figures 10, 11 and 12, in an exemplary embodiment, the arms 101.2 may include ramped proximal ends 101.1.2.2. The proximal end 101.1.2.2 is adapted to, in use, engage a latch element 101.2.2 arranged on a distal end of the needle hub 101.2, as described further below. In an exemplary embodiment, the latch element 101.2.2 may be designed as T-shaped radial protrusion having a cross width substantially equal to the second width W102 and greater than the first and third widths W101, W103 and a stem width substantially equal to the first and third widths W101, W103.

In the exemplary embodiment, the needle hub 101.2 comprises a thread 101.2.3 for mounting the needle safety device 101 to a not shown medicament delivery device. Alternatively, the needle hub 101.2 may be attached to the delivery device by other suitable couplings, like for example bayonet type couplings or snap-fit couplings. The delivery device can be designed as a pen injector or according to alternative embodiments, the delivery device may be designed as a syringe, a dental syringe, an auto-injector or a similar device suitable for delivering a medicament to a patient.

The needle 101.3 is mounted to the needle hub 101.2. In the exemplary embodiment shown, the needle 101.3 is arranged as a double pointed needle with a pointed distal tip 101.3.1 and a pointed proximal tip 101.3.2. The pointed proximal tip 101.3.2 is adapted to be inserted into a cartridge or container containing the medicament that is retained in the delivery device when the needle safety device 101 is coupled to the delivery device. In an exemplary embodiment, the biasing element 101.4 is adapted to bias the needle shield 101.1 with respect to the needle hub 101.2 in a first axial position and arranged as a compression spring which bears against the needle shield 101.1 in the distal direction D and against the needle hub 101.2 in a proximal direction P. That is, in the first axial position, the biasing element 101.4 may be preloaded. In another exemplary embodiment, there may be no load on the biasing element 101.4 in the first axial position.

In this exemplary embodiment, the latch element 101.2.2 may also act as a retaining element to prevent distal movement of the needle shield 101.1 relative to the needle hub 101.2 prior to use. For example, the latch element 101.2.2 may abut a proximal portion of the needle shield 101.1 which is proximal of the latch element 101.2.2.

Figure 13 shows the exemplary embodiment of Figure 10 in a first axial position (PA101) in which the needle shield 101.1 covers the distal tip 101.3.1 of the needle 101.3. In the first axial position (PA1), the proximal end 101.1.2.2 of the arm 101.1.2 may cover the latch element 101.2.2.

Figure 14 shows the exemplary embodiment of Figure 10 when the needle safety device 101 is pressed against an injection site. As the needle shield 101.1 moves proximally relative to the needle hub 101.2, the latch element 101.2.2 engages the proximal end 101.1.2.2 of the arm 101.1.2, causing the arm 101.1.2 to deflect radially. When the latch element 101.2.2 reaches the second section 101.S2, the latch element 101.2.2 will project through the second section 101.S2 because the cross section of the latch element 101.2.2 is substantially equal to the second width W2 and the arm 101.1.2 returns to its non-deflected state. As the needle 101.1 moves further proximally relative to the needle hub 101.2, the latch element 101.2.2 engages the first section 101.S1.

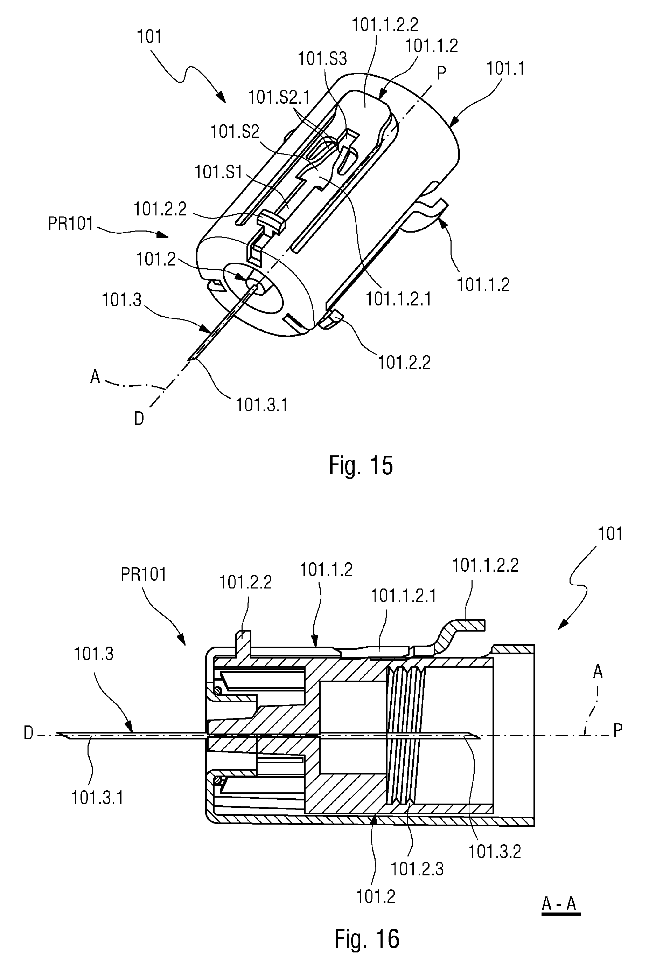

Figures 15 and 16 show the exemplary embodiment of the needle safety device 101 in a retracted position (PR101) in which the needle shield 101.1 has moved proximally relative to the needle hub 101.2 such that the distal tip 101.3.1 of the needle 101.3 is exposed. The needle safety device 101 may attain the retracted position (PR101) when the needle safety device 101 is pressed against an injection site. In the retracted position (PR10), the biasing element 101.4 may be compressed. In the retracted position (PR101), the latch element 101.2.2 is in the first section 101.S1.

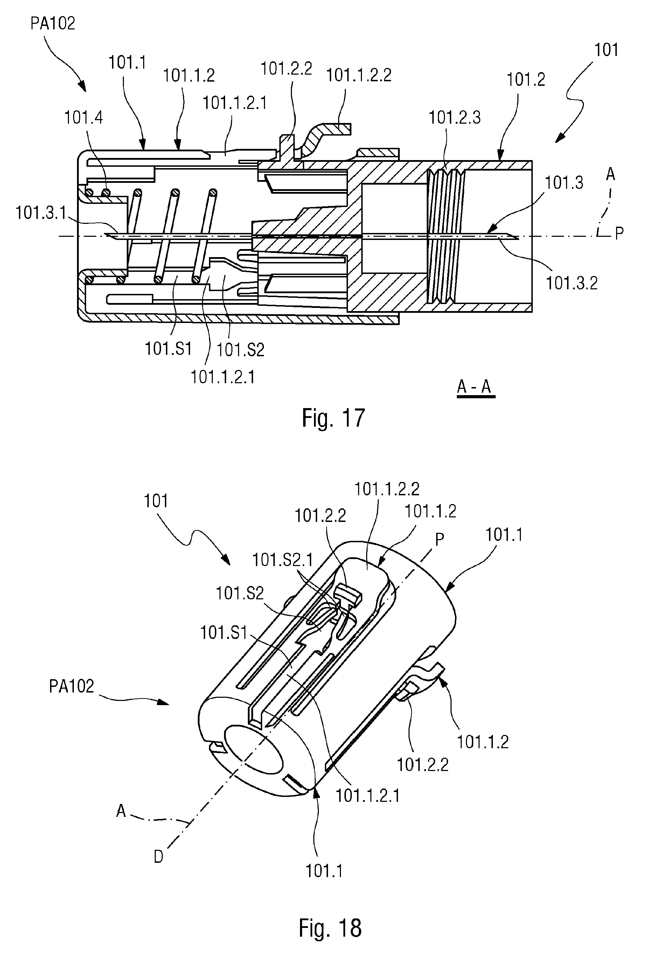

Figures 17 and 18 show the exemplary embodiment of the needle safety device 101 in a second axial position (PA102) in which the needle shield 101.1 has moved distally relative to the needle hub 101.2 such that the distal tip 101.3.1 of the needle 101.3 is covered. When the needle shield 101.1 moves distally from the retracted position (PR101), under the force of the biasing element 101.4, the latch element 101.2.2 passes proximally through the first section 101.S1 and the second section 101.S2. When moving from the second section 101.S2 to the third section 101. S3, the latch element 101.2.2 engages the stop elements 101.S2.1, causing the stop elements 101.S2.1 to deflect radially relative to the axis of the aperture 101.1.2.1 and allowing the latch element 101.2.2 to engage the third section 101.S3. When the latch element 101.1.2.2 has engaged the third section 101.S3, the stop elements 101.S2.1 return to their non-deflected position locking the latch element 101.1.2.2 in the third section 101.S3. That is, the stop elements 101.S2.1 abut the latch element 101.1.2.2 if the needle shield 101.1 is attempted to move proximally relative to the needle hub 101.2.

Hence the engagement of the latch element 101.2.2 and the third section 101.S3 and the stop elements 101.S2.1 introduces a no-return feature so that needle safety device 101 may only be used once and needle stick injuries with contaminated needles may be avoided. The safety needle device 101 according to the first embodiment is irreversibly locked in the second advanced position (PA102) and may be safely detached from the delivery device and disposed after use.

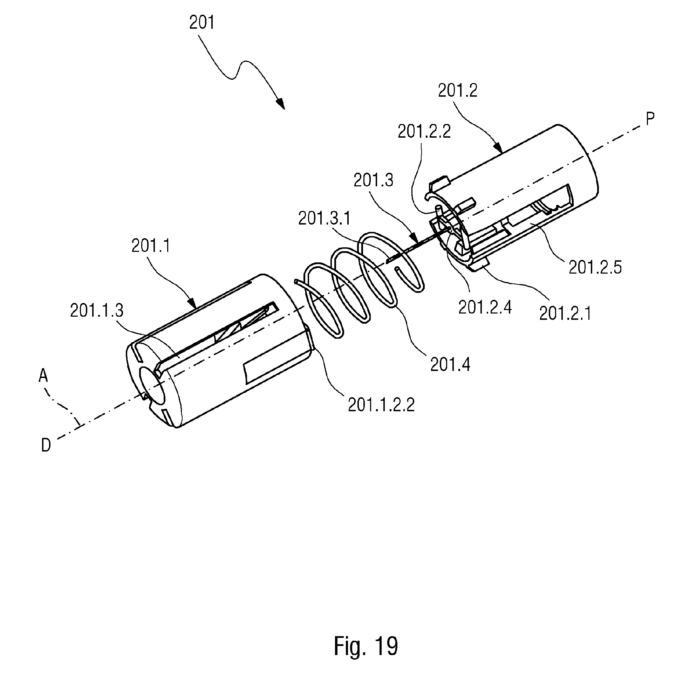

Figure 19 shows yet another exemplary embodiment of a needle safety device 201 according to the present invention. The needle safety device 201 comprises a needle shield 201.1 telescopically coupled to a needle hub 201.2 having a needle 201.3, and a biasing element 201.4 applying a biasing force to the needle shield 201.1 relative to the needle hub 201.2. Further, the needle safety device 201 includes a longitudinal axis A extending through from a proximal direction P to a distal direction D.

In an exemplary embodiment, the needle shield 201.1 includes one or more slots 201.1.3 adapted to engage retaining elements 201.2.1 formed on a distal end of the needle hub 201.2. In use, as described further below, the retaining elements 201.2.1 translate within the slots 201.1.3 to prevent removal of the needle shield 201.1 from the needle hub 201.2, limit movement of the needle shield 201.1 relative to the needle hub 201.2 in a proximal direction, and prevent rotation of the needle shield 201.1 relative to the needle hub 201.2.

The needle 201.3 is coupled to the needle hub 201.2 via a stem 201.2.4 formed on the needle hub 201.2. Extending radially from the stem 201.2.4 may be one or more latch elements 201.2.2. In an exemplary embodiment, the latch elements 201.2.2 may be T-shaped having a stem portion extending radially from the stem 201.2.4, and a cross portion extending perpendicular to the stem portion.

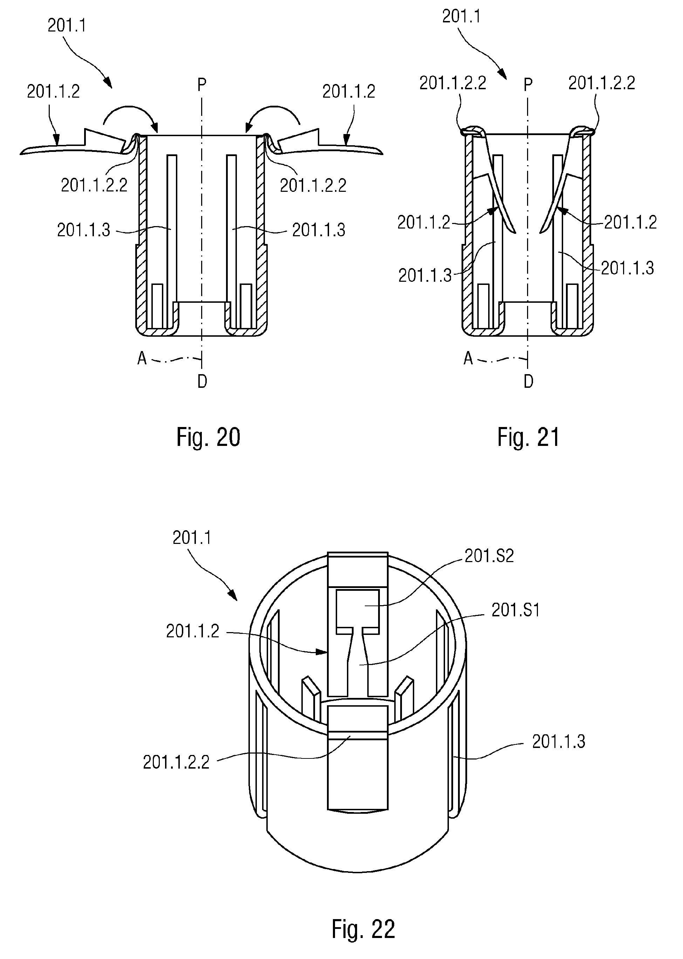

Figures 20, 21 and 22 show an exemplary embodiment of the needle shield 201.1. The needle shield 201.1 includes two arms 201.1.2 coupled to a proximal end of the needle shield 201.1 via hinges 201.1.2.2. The hinges 201.1.2.2 may be formed as a part of the arms 201.1.2 e.g. The hinges 201.1.2.2 may be made from another softer and/or thinner material than the other part of the arms 201.1.2. Figure 20 shows the arms 201.1.2 in an open position and Figures 21 and 22 show the arms 201.1.2 in a closed position, within the needle shield 201.1. In the closed position, the arms 201.1.2 are resiliently biased against the needle shield 201.1 by abutment members. The abutment members allow the arms 201.1.2 to deflect radially when pressure is applied to the arms 201.1.2. The needle hub 201.2 may include channels 201.2.5 for accommodating the arms 201.1.2. In the closed position, the arms 201.1.2 may be disposed at an angle towards the axis A.

Figure 22 also shows that the arms 201.1.2 include an aperture 201.1.2.1 adapted to engage the latch element 201.2.2 on the stem 201.2.4 of the needle hub 201.2. In an exemplary embodiment, the aperture 201.1.2.1 includes a first distal section 201.S1 having a first width W201 and a second proximal section 201.S2 having a second width W202, greater than the first width W201.

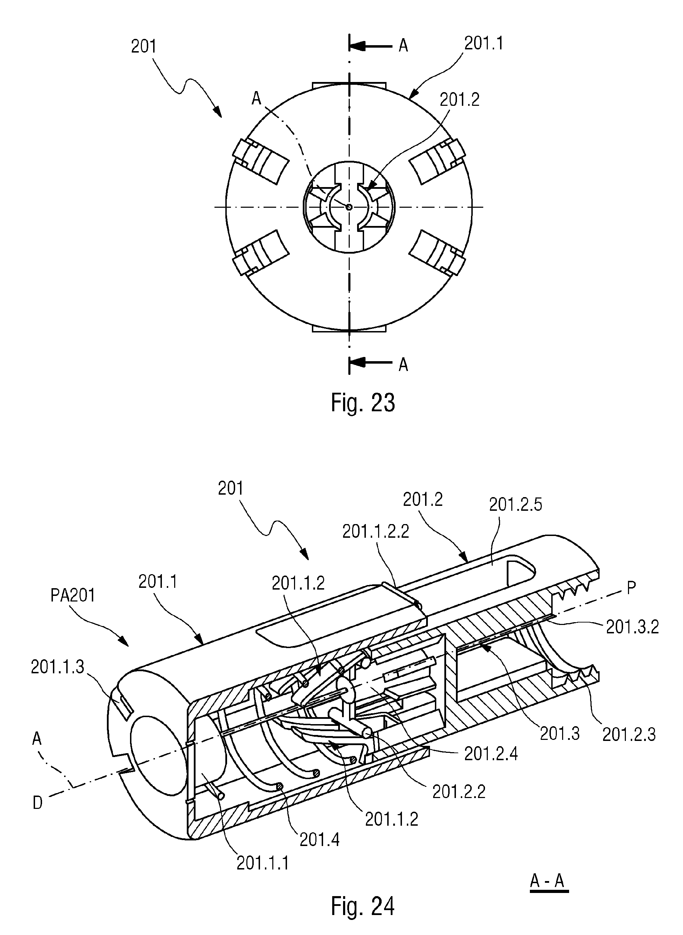

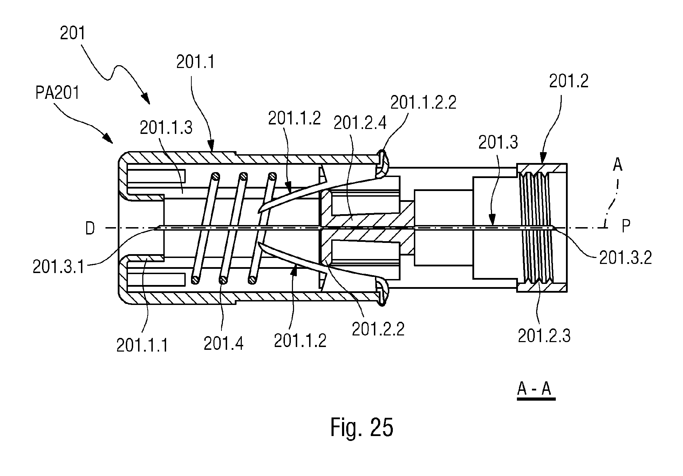

Figures 23, 24 and 25 show the exemplary embodiment of the needle safety device 1 shown in Figure 19 in a first axial position (PA201). In the exemplary embodiment, the needle hub 201.2 comprises a thread 201.2.3 for mounting the needle safety device 201 to a not shown medicament delivery device. Alternatively, the needle hub 201.2 may be attached to the delivery device by other suitable couplings, like for example bayonet type couplings or snap-fit couplings. The delivery device can be designed as a pen injector or according to alternative embodiments, the delivery device may be designed as a syringe, a dental syringe, an auto-injector or a similar device suitable for delivering a medicament to a patient.

The needle 201.3 is mounted to the needle hub 201.2. In the exemplary embodiment shown, the needle 201.3 is arranged as a double pointed needle with a pointed distal tip 201.3.1 and a pointed proximal tip 201.3.2. The pointed proximal tip 201.3.2 is adapted to be inserted into a cartridge or container containing the medicament that is retained in the delivery device when the needle safety device 201 is coupled to the delivery device.

In the first axial position (PA201), the needle shield 201.1 is in an advanced axial position relative to the needle hub 201.2 such that the needle shield 201.1 covers the distal tip 201.3.1 of the needle 201.3. The needle shield 201.1 is prevented from moving proximally relative to the needle hub 201.2 when in the first axial position (PA201) by the retaining elements 201.2.1 which abut a proximal end of the slots 201.1.3.

In an exemplary embodiment, the biasing element 201.4 is adapted to bias the needle shield 201.1 with respect to the needle hub 201.2 in the first axial position (PA201) and arranged as a compression spring which bears against the needle shield 201.1 in the distal direction D and against the needle hub 201.2 in a proximal direction P. That is, in the first axial position (PA201), the biasing element 201.4 may be preloaded. In another exemplary embodiment, there may be no load on the biasing element 201.4 in the first axial position (PA201).

In the first axial position (PA201), the latch elements 201.2.2 may engage the first section 201.S1 of the arms 201.1.2. In an exemplary embodiment, in the first axial position (PA201), the latch elements 201.2.2 may apply a load on the arms 201.1.2 such that the arms 201.1.2 are deflected radially. In another exemplary embodiment, the in the first axial position (PA201), the latch elements 201.2.2 may abut the arms 201.1.2 but not exert any force on the arms 201.1.2.

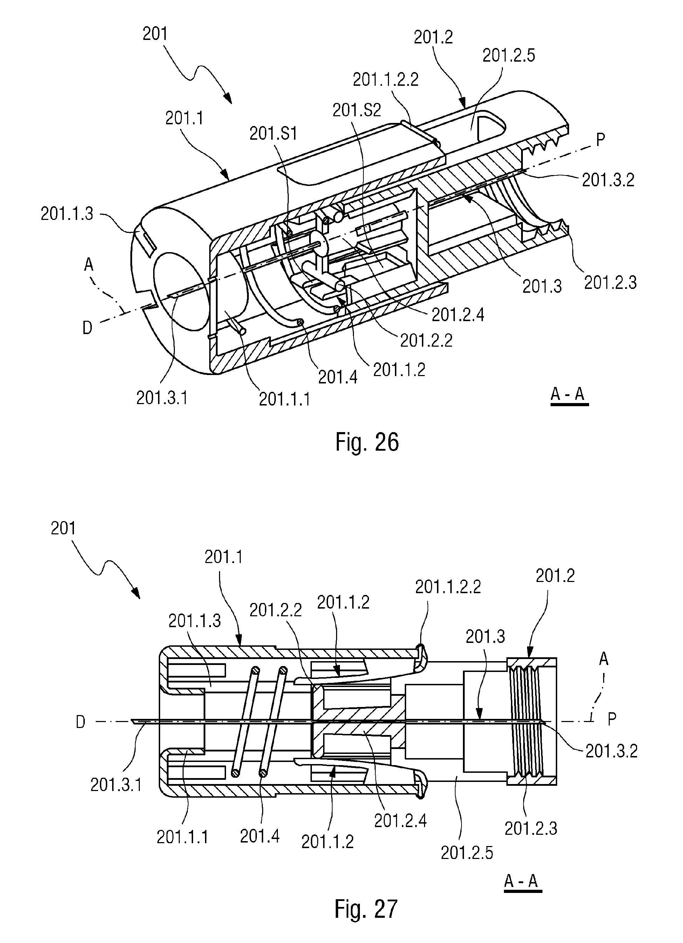

Figures 26 and 27 shows the exemplary embodiment of Figure 19 when the needle safety device 201 is pressed against an injection site. As the needle shield 201.1 moves proximally relative to the needle hub 201.2, the latch element 201.2.2 engages the arm 201.1.2, causing the arm 201.1.2 to deflect radially.

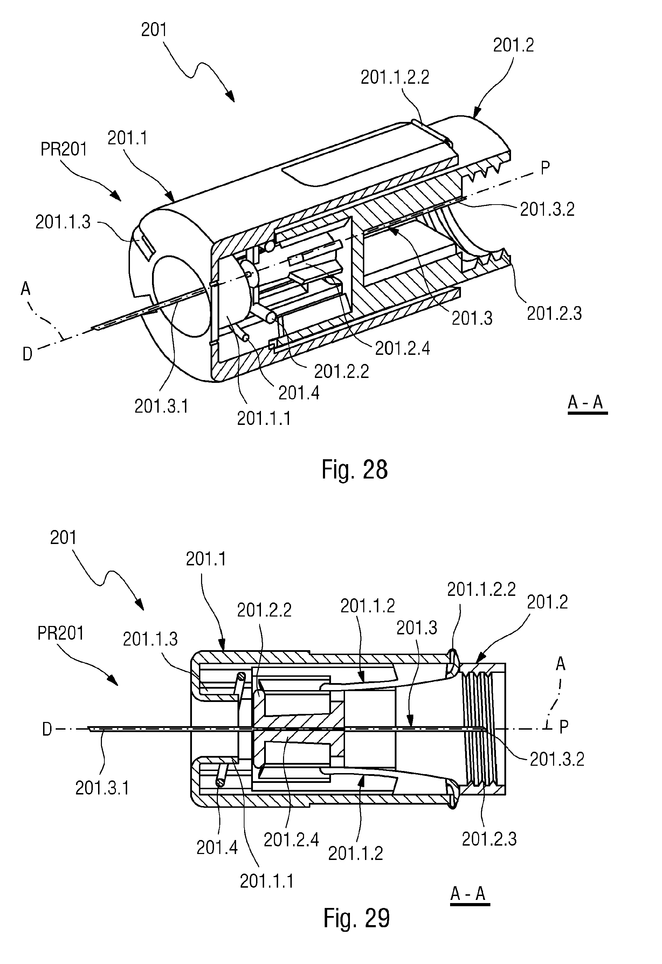

Figures 28 and 29 show the exemplary embodiment of the needle safety device 201 in a retracted position (PR201) in which the needle shield 201.1 has moved proximally relative to the needle hub 201.2 such that the distal tip 201.3.1 of the needle 201.3 is exposed. The needle safety device 201 may attain the retracted position (PR201) when the needle safety device 201 is pressed against an injection site. In the retracted position (PR201), the biasing element 201.4 may be compressed. In the retracted position (PR201), the latch elements 201.2.2 may have bypassed distal ends of the arms 201.1.2, allowing the arms 201.1.2 to return to a non-deflected position. In another exemplary embodiment, the arms 201.1.2 may abut the stem 201.2.4 or other portion of the needle hub 201.1, remaining in the deflected position.

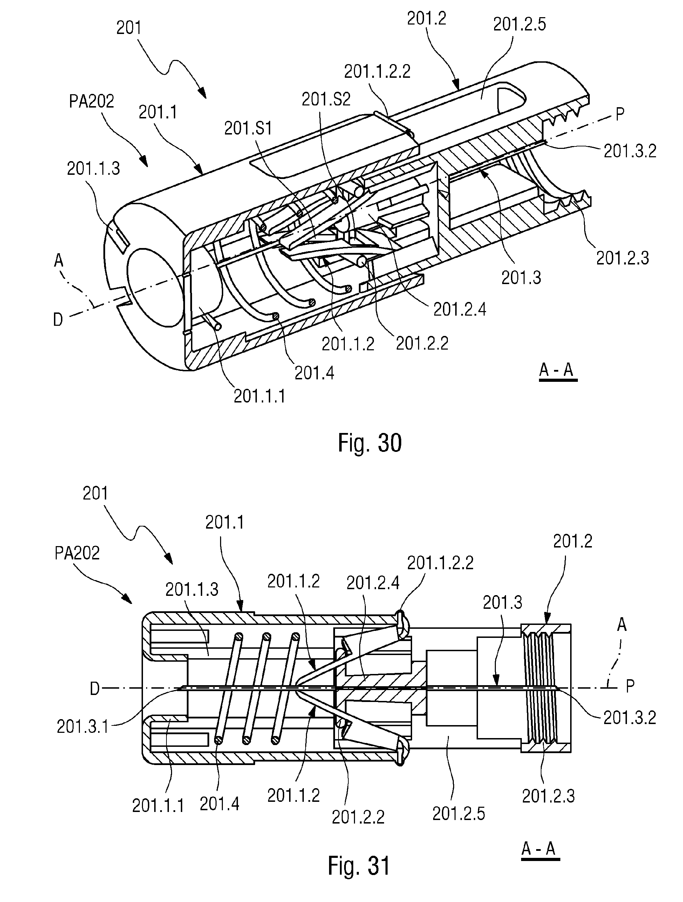

Figures 30 and 31 the exemplary embodiment of the needle safety device 201 in a second axial position (PA202) in which the needle shield 201.1 has moved distally relative to the needle hub 201.2 such that the distal tip 201.3.1 of the needle 201.3 is covered. When the needle shield 201.1 moves distally from the retracted position (PR201), under the force of the biasing element 201.4, the arms 201.1.2 disengage the stem 201.2.4 and other portion of the needle hub 201.1 and return to the non-deflected position. Thus, as the needle shield 201.1 moves distally, the latch elements 201.2.2 engage the apertures 201.1.2.1 of the arms 201.2. The latch elements 201.2.2 pass proximally through the first section 201.S1 and the second section 201.S2. The latch elements 201.2.2 may engage stop elements between the first section 201.S1 and the second section 201.S2 which causes the arms 201.1.2 to deflect and allow the latch element 201.2.2 to engage the second section 201.S2. When the latch element 201.1.2.2 has engaged the second section 201.S2, the stop elements return to their non-deflected position locking the latch element 201.1.2.2 in the second section 201.S2. That is, the stop elements abut the latch element 201.1.2.2 if the needle shield 201.1 is attempted to move proximally relative to the needle hub 201.2.

Hence the engagement of the latch element 201.2.2 and the second section 201.S2 and the stop elements introduces a no-return feature so that needle safety device 201 may only be used once and needle stick injuries with contaminated needles may be avoided. The safety needle device 201 according to the first embodiment is irreversibly locked in the second advanced position (PA202) and may be safely detached from the delivery device and disposed after use.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A needle safety device (1, 101, 201) comprising:
a needle hub (1.2, 101.2, 201.2) including a latch element (1.2.2, 101.2.2, 201.2.2);
a needle (1.3, 101.3, 201.3) coupled to the needle hub (1.2, 101.2, 201.2), the needle (1.3, 101.3, 201.3) having a distal tip (1.3.1, 101.3.1, 201.3.1);
a needle shield (1.1, 101.1, 201.1) telescopically coupled to the needle hub (1.2, 101.2, 201.2), the needle shield (1.1, 101.1, 201.1) including a resilient arm (1.1.2, 101.1.2, 201.1.2) adapted to engage the latch element (1.2.2, 101.2.2, 201.2.2),
wherein, when the needle shield (1.1, 101.1, 201.1) is in a first axial position (PA1, PA101, PA201) relative to the needle hub (1.2, 101.2, 201.2), the needle shield (1.1, 101.1, 201.1) covers the distal tip (1.3.1, 101.3.1, 201.3.1) of the needle (1.3, 101.3, 201.3),
wherein, when the needle shield (1.1, 101.1, 201.1) is in a retracted position (PR1, PR101, PR201) relative to the needle hub (1.2, 101.2, 201.2), the needle shield (1.1, 101.1, 201.1) is retracted to expose the distal tip (1.3.1, 101.3.1, 201.3.1) of the needle (1.3, 101.3, 201.3) and the arm (1.1.2, 101.1.2, 201.1.2) is deflected radially, and
wherein, when the needle shield (2.1) is in a second axial position (PA2) relative to the needle hub (1.2, 101.2, 201.2), the needle shield (1.1, 101.1, 201.1) covers the distal tip (1.3.1, 101.3.1, 201.3.1) of the needle (1.3, 101.3, 201.3) and the arm (1.1.2, 101.1.2, 201.1.2) engages the latch element (1.2.2, 101.2.2, 201.2.2) to prevent proximal movement of the needle shield (1.1, 101.1, 201.1) relative to the needle hub (1.2, 101.2, 201.2).

2. The needle safety device (1, 201) according to claim 1, wherein the needle hub (1.2, 201.2) includes a retaining element (1.2.1, 201.2.1) adapted to engage a slot (1.1.3, 201.1.3) on the needle shield (1.1, 201.1) to prevent distal movement of the needle shield (1.1, 2010.1) relative to the needle hub (1.2, 201.2) in the first axial position (PA1, PA201).

3. The needle safety device (1) according to any of the preceding claims, wherein the needle shield (1.1) includes a cover element (1.1.1) defining a cavity for accommodating deflection of the arm (1.1.2).

4. The needle safety device (1) according to any of the preceding claims, wherein the latch element (1.2.2) includes a resilient guide (1.2.2.2) formed in a recess (1.2.2.1).

5. The needle safety device (1) according to claim 4, wherein a proximal end of the arm (1.1.2) includes a hook (1.1.2.1) having a first ramped surface adapted to deflect the guide (1.2.2.2) when the needle shield (1.1) moves from the retracted position (PR) to the second axial position (PA2).

6. The needle safety device (1) according to claim 5, wherein, when the guide (1.2.2.2) is deflected, the hook (1.1.2.1) engages the recess (1.2.2.1) to lock the needle shield (1.1) in the second axial position (PA2).

7. The needle safety device (101, 201) according to claim 1, wherein the arm (101.1.2, 201.1.2) includes an aperture (101.1.2.1, 201.1.2.1) adapted to engage the latch element (101.2.2, 201.2.2).

8. The needle safety device (101, 201) according to claim 7, wherein the aperture (101.1.2.1, 201.1.2.1) includes a first section (101.S1, 201.S1) having a first width (W101) and a second section (101.S2, 201.S2) having a second width (W102).

9. The needle safety device (101) according to claim 8, wherein a third section (101.S3) having a third width (W103), wherein the first width (W101) and the third width (W103) are substantially equal.

10. The needle safety device (101) according to claim 9, wherein resilient stop elements (101.S2.1) are formed between the second section (101.S2) and the third section (101.S3).

11. The needle safety device (101, 201) according to claim 8, wherein the latch element (101.2.2, 201.2.2) is T-shaped and has a cross section substantially equal to the second width (W102) and a stem section substantially equal to the first width (W101).

12. The needle safety device (101, 201) according to claim 11, wherein the cross section of the latch element (101.2.2, 201.2.2) engages the arm (101.1.2, 201.1.2) and causes the arm (101.1.2, 201.1.2) to deflect radially when the needle shield (101.1, 201.1) is moved from the first axial position (PA101, PA201) to the retracted position (PR101, PR201).

13. The needle safety device (101) according to claim 12, wherein the latch element (101.2.2) engages the aperture (101.1.2.1) when the cross section reaches the second section (101.S2) when the needle shield (101.1) is moved from the first axial position (PA101) to the retracted position (PR101).

14. The needle safety device (101) according to claim 13, wherein the stem portion causes the stop elements (101.S2.1) to deflect when the needle shield (101.1) moves from the retracted position (PR101) to the second axial position (PA102).

15. The needle safety device (101) according to claim 14, wherein, when the latch element (101.2.2) is in the third section (101.S3), the stop elements (101.S2.1) return to a non-deflected position to lock latch element (101.2.2) in the third section (101.S3) and lock the needle shield (101.1) in the second axial position (PA102).

16. The needle safety device (201) according to claim 11, wherein the cross section abuts the arm (201.1.2) in the first axial position (PA201).

17. The needle safety device (201) according to claim 16, wherein the latch element (201.2.2) engages the aperture (201.1.2.1) when needle shield (201.1) is moved from the retracted position (PR201) to the second axial position (PA202).

18. The needle safety device (201) according to claim 17, wherein the stem portion causes a narrowed portion of the aperture (201.1.2.1) to deflect when latch element (201.2.2) moves from the first section (201.S1) to the second section (201.S2).

19. The needle safety device (201) according to claim 18, wherein, when the latch element (201.2.2) is in the second section (201.S2), the narrowed portion of the aperture (201.1.2.1) returns to a non-deflected position to lock latch element (201.2.2) in the second section (201.S2) and lock the needle shield (201.1) in the second axial position (PA202).

20. The needle safety device (1, 101, 201) according to any of the preceding claims, further comprising:
a biasing element (1.4, 101.4, 201.4) applying a biasing force to the needle shield (1.1, 101.1, 201.1) in a distal direction when the needle shield (1.1, 101.1, 201.1) is in the retracted position (PR1, PR101, PR201).
